Europäisches Patentamt

European Patent Office

Office européen des brevets

(11) Publication number: **0 492 447 A1**

# EUROPEAN PATENT APPLICATION

(21) Application number: **91121802.2**

(22) Date of filing: **19.12.91**

(51) Int. Cl.5: **C12N 9/02**, C12P 21/00

(30) Priority: **28.12.90 JP 416786/90**

(43) Date of publication of application:
**01.07.92 Bulletin 92/27**

(84) Designated Contracting States:
**DE FR GB IT SE**

(71) Applicant: **TOYO JOZO CO., LTD.**
**632-1, Mifuku Ohito-cho**
**Tagata-gun Shizuoka-ken(JP)**

(72) Inventor: **Mizoguchi, Junzo**
**357-8, Ohitocho-Mikado**
**Tagata-gun, Shizuoka 410-23(JP)**
Inventor: **Otani, Tsuchio**
**7-3, Minamicho**
**Mishima-shi, Shizuoka 411(JP)**
Inventor: **Katayama, Kazuhiko**
**3-5, Nakatamachi**
**Mishima-shi, Shizuoka 411(JP)**
Inventor: **Yamashita, Tomoyuki**
**980, Yawatano**
**Itoh-shi, Shizuoka 413-02(JP)**
Inventor: **Nagoshi, Hisaki**
**883, Ohitocho-Mifuku**
**Tagata-gun, Shizuoka 410-23(JP)**
Inventor: **Akashi, Hirotada**
**Nr. 303, Mochizuki Mansion, 239-1, Ichoda**
**Mishima-shi, Shizuoka 411(JP)**

(74) Representative: **Modiano, Guido et al**
**MODIANO, JOSIF, PISANTY & STAUB**
**Baaderstrasse 3**
**W-8000 München 5(DE)**

(54) **Process for preparing active extracellular superoxide dismutase.**

(57) Active EC-SOD is produced by subjecting insoluble, inactive aggregates containing recombinant EC-SOD produced by transformed bacteria or the like to (a) a step for treating said inactive forms of recombinant foreign proteins containing EC-SOD in a chaotropic conditions under reducing conditions or in combination of the two, and forming and recovering a solubilized monomeric EC-SOD, (b) a step for refolding said monomer into an active EC-SOD under a refolding condition, and (c) a step for purifying the refolded active EC-SOD.

The EC-SOD is useful as an anti-inflammatory agent or a medical for protecting Behcet disease, myocardiac infraction, radiation disorders or the like.

EP 0 492 447 A1

## FIG. 1-1

```
CTAGAGGGTTATTAATAATGTGGACTGGTGAAG
  TCCCAATAATTATTACACCTGACCACTTC

ATTCTGCTGAACCGAACTCTGATTCTGCTGAAT
TAAGACGACTTGGCTTGAGACTAAGACGACTTA

GGATCCGTGATATGTACGCTAAAGTAACTGAAA
CCTAGGCACTATACATGCGATTTCATTGACTTT

TCTGGCAGGAAGTTATGCAGCGTCGTGATGACG
AGACCGTCCTTCAATACGTCGCAGCACTACTGC

ATGGTACTCTGCATGCTGCTTGTCAGGTTCAGC
TACCATGAGACGTACGACGAACAGTCCAAGTCG

CGTCTGCTACTCTGGACGCTGCACAGCCACGTG
GCAGACGATGAGACCTGCGACGTGTGGGTGCAC

TTACCGGTGTTGTTCTGTTCCGTCAGCTGGCAC
AATGGCCACAACAAGACAAGGCAGTCGACCGTG

CACGTGCTAAACTGGATGCTTTCTTCGCTCTGG
GTGCACGATTTGACCTACGAAAGAAGCGAGACC

AAGGCTTCCCGACTGAACCGAACTCTTCCTCTC
TTCCGAAGGGCTGACTTGGCTTGAGAAGGAGAG

GTGCTATCCATGTTCATCAGTTCGGTGATCTGT
CACGATAGGTACAAGTAGTCAAGCCACTAGACA

CTCAGGGTTGTGAATCTACCGGTCCACATTACA
GAGTCCCAACACTTAGATGGCCAGGTGTAATGT
```

## FIG. 1-2

```
ACCCGCTGGCTGTTCCGCACCCGCAGCATCCGG
TGGGCGACCGACAAGGCGTGGGCGTCGTAGGCC

GTGACTTCGGTAACTTCGCGGTTCGTGACGGCT
CACTGAAGCCATTGAAGCGCCAAGCACTGCCGA

CTCTGTGGCGTTACCGTGCTGGTCTGGCTGCTT
GAGACACCGCAATGGCACGACCAGACCGACGAA

CTCTGGCGGGTCCGCATTCCATCGTTGGCCGTG
GAGACCGCCCAGGCGTAAGGTAGCAACCGGCAC

CTGTTGTTGTTCACGCTGGCGAAGATGACCTGG
GACAACAACAAGTGCGACCGCTTCTACTGGACC

GTCGTGGTGGTAACCAGGCTTCTGTTGAAAACG
CAGCACCACCATTGGTCCGAAGACAACTTTTGC

GTAACGCAGGCCGTCGTCTGGCTTGTTGCGTTG
CATTGCGTCCGGCAGCAGACCGAACAACGCAAC

TTGGCGTTTGCGGTCCAGGTCTGTGGGAACGTC
AACCGCAAACGCCAGGTCCAGACACCCTTGCAG

AGGCACGTGAACATTCTGAACGTAAAAAACTGC
TCCGTGCACTTGTAAGACTTGCATTTTTTGACG

GTCGTGAATCCGAATGCAAAGCAGCTTGACAAG
CAGCACTTAGGCTTACGTTTCGTCGAACTGTTC

TTAA
```

2

EP 0 492 447 A1

The present invention relates to a process for preparing active extracellular superoxide dismutase (referred to hereinafter as EC-SOD). More particularly, the present invention relates to a process for efficiently obtaining ensymatically active EC-SOD from insoluble inactive aggregates containing recombinant EC-SOD formed within hosts such as bacteria by a recombinant DNA technique.

The EC-SOD obtained by the process of the present invention is effective to arthritis or rheumatism as an anti-inflammatory agent, and it is also expected to be effective to Behcet's disease, ischemic cardiac disorders such as myocardial infraction, collagen disease, radiation disorders or the like.

Superoxide dismutase (referred to hereinafter as SOD) is also called a superoxide scavenger enzyme and present in organisms which consume oxygen, and is known to have an enzymatic activity for specifically eliminating superoxide anion radicals (active oxygen: referred to as $O_2^-$) by the by the mechanism shown in the following equation:

$$2O_2^- + 2H^+ \rightarrow O_2 + H_2O_2$$

The enzyme is present in a wide range of organisms from microorganisms as lower organisms to higher animals or plants and acts protectively on active oxygen disorders in organisms.

Active oxygen is generated in phagocyte such as macrophage and plays a role to decompose the foreign substances caught by the phagocyte. If the active oxygen is produced excessively, it is released extracellularly and causes lesions at the other tissues. The SOD decomposes the excessive active oxygen which causes lesions and protects the tissues, so that it is effective as an anti-inflammatory agent to arthritis or rheumatism and is expected to have effectiveness for the prevention of Behcet's disease, ischemic cardiac disorders such as myocardiac infraction, collagen disease, radiation disorders or the like.

As the types of SOD, Cu Zn-SOD localized in the soluble fraction of cell and Mn-SOD in mitochondria are known. In addition, EC-SOD which occurs in human lung tissue and has physicochemical properties different from the conventionally known SODs has been recently discovered (Proc. Nat. Acad. Sci. USA, 79, 7634-7638 (1982)).

The EC-SOD is a glycoprotein which is constituted from four subunits and has a sugar chain having a molecular weight of about 135,000 dalton and has four copper and four zinc ions per molecule. The cDNA of the enzyme has been cloned from human placental tissue (Proc. Nat. Acad. Sci. USA, 84, 6340-6344 (1987)), and the expression of the EC-SOD gene by CHO cells with the cDNA has been tried (Proc. Nat. Acad. Sci. USA, 84, 6634-6638 (1987); Japanese Patent Publication No. 501473/1988).

In the expression process of the EC-SOD gene in an animal cell such as a CHO cell as a host, however, there was a problem that uniform EC-SOD could not be produced in a large scale due to the little amount of EC-SOD expressed and the ununiformity of sugar chains added.

As a method for solving the problem, the use of an expression system with bacteria such as Escherichia coli as a host is expected. The EC-SOD is, however as described above, a glycoprotein of very complicated structure which is a tetramer containing copper and zinc ions and has a sugar chain. Thus, it is very difficult to express an active type of EC-SOD by the use of E. coli as a host, and there has hitherto been found no method for preparing enzymatically active EC-SOD with use of bacteria as a host.

Under the circumstances, a process is desired for producing efficiently enzymatically active EC-SOD with an expression system with a microorganism such as a bacterium as a host.

The present inventors have conducted earnest research in order to reach the aforementioned purpose. First, as the EC-SOD gene, a completely new gene as shown in the sequence number 1 in the sequence table was made by total synthesis to construct an expression vector, which is next integrated into a bacterium such as E. coli in place of the conventionally employed CHO cell to express the EC-SOD gene. They have found that, while the EC-SOD protein is expressed, insoluble inactive aggregates containing recombinant EC-SOD having no enzymatic activity are produced in the cell.

The present inventors have found as a result of further research that EC-SOD having high enzymatic activity of 3000 unit/mg or more is successfully obtained by treating the insoluble inactive aggregates containing recombinant EC-SOD under a particular condition to form the EC-SOD as a solubilized monomer, which is refolded into active EC-SOD under a refolding condition and recovered by the refolded crude active EC-SOD, and the aforementioned purpose is achieved. Thus, the present invention has been accomplished based on this knowledge.

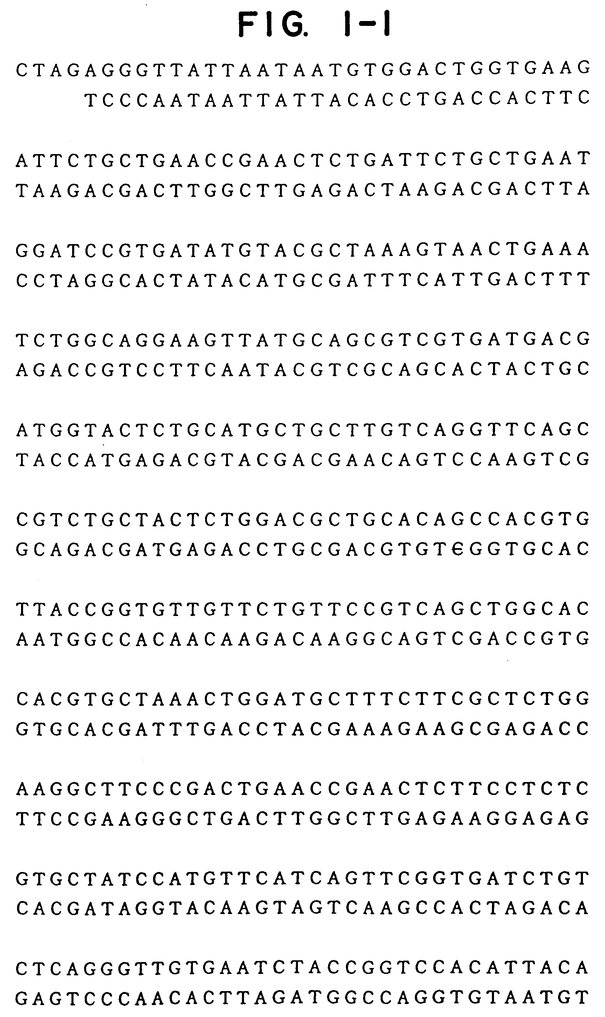

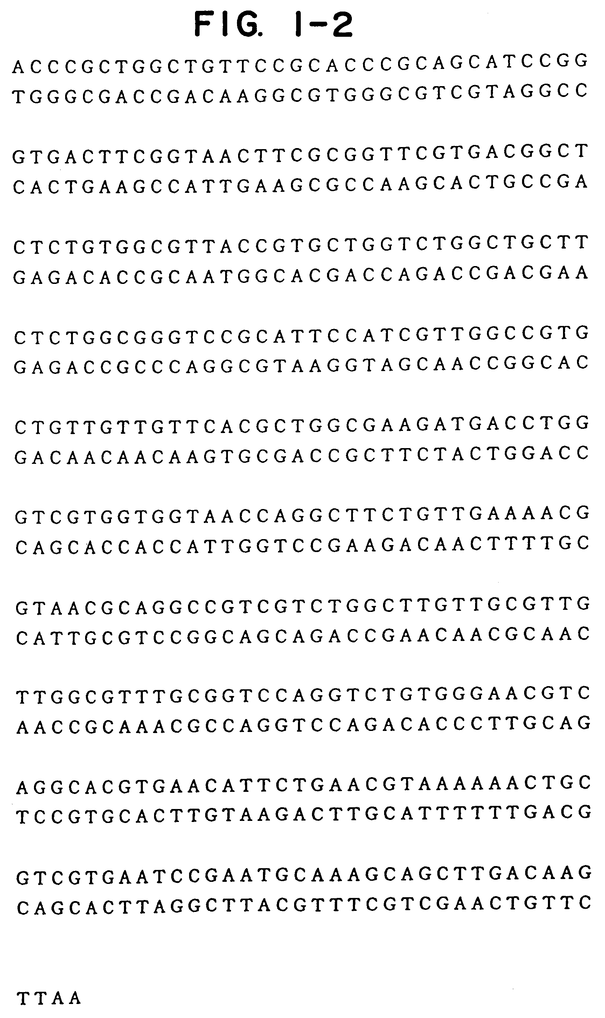

In the accompanying drawings, Fig. 1-1 illustrates the first half portion of the base sequence of the synthetic DNA for the EC-SOD gene used for the expression vector,

Fig. 1-2 illustrates the latter half of the base sequence of the synthetic DNA for the EC-SOD gene used for the expression vector, in which TTAA at the last part of Fig. 1-2 is the lower part of the base sequence,

3

EP 0 492 447 A1

Fig. 2 illustrates the base sequence of 15 oligonucleotides for the synthesis of DNA illustrated in Figs. 1-1 and 1-2, and

Fig. 3 illustrates the flow chart for constructing the EC-SOD expression vector used in the present invention.

According to the present invention, a process for producing enzymatically active purified EC-SOD from insoluble inactive aggregates containing recombinant EC-SOD is provided, wherein the following steps are sequentially performed: (a) the insoluble inactive aggregates containing recombinant EC-SOD is treated under a chaotropic environment or a reducing condition or in combination of the two to form the EC-SOD as a solubilized monomer and to recover it; (b) the monomeric EC-SOD recovered in the step (a) is refolded into active EC-SOD under a refolding condition; and (c) the active EC-SOD refolded in the step (b) is purified.

According to the process of the present invention, highly purified EC-SOD having an enzymatically high activity is obtained efficiently from insoluble and inactive aggregates containing recombinant EC-SOD which are produced in the cells of host microorganisms such as a bacterium transformed by the transduction of the EC-SOD gene. Accordingly, the process of the present invention is industrially valuable as a technique for the large scale production of highly active EC-SOD by the gene recombination of microorganisms such as a bacterium.

The present invention is now described in detail below.

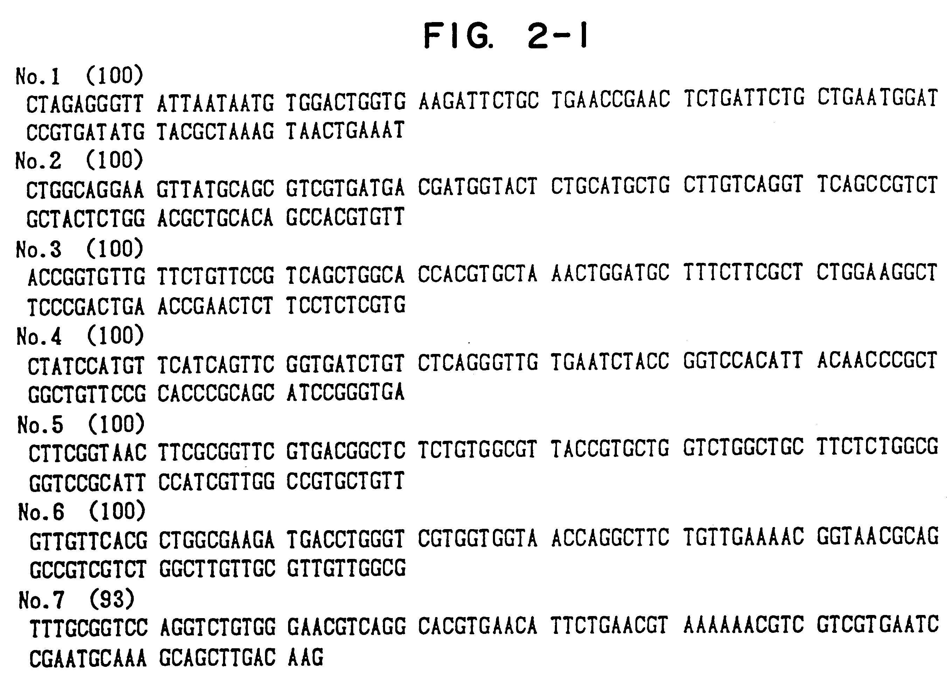

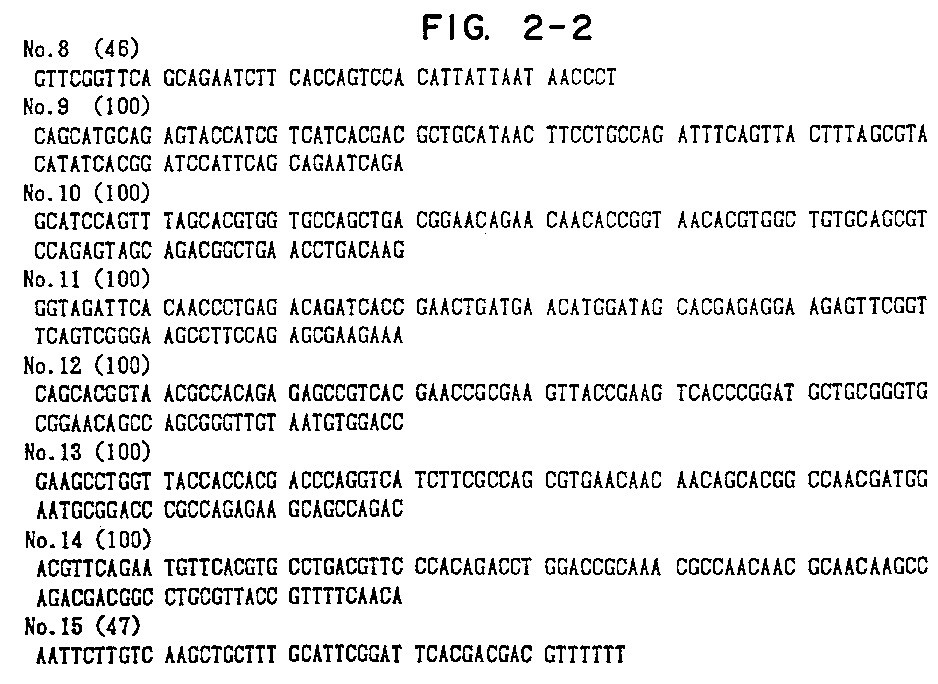

In the process of the present invention, a method for expressing the EC-SOD gene transduced into a bacterium such as E. coli as a host is used, and thus it is necessary first to construct an expression vector to be introduced into the bacterium with the EC-SOD gene. In the present invention, a synthetic double-stranded straight chain DNA having a base sequence shown in Figs. 1-1 and 1-2 is used for the EC-SOD gene for constructing the expression vector. The DNA may be synthesized for example by synthesizing 15 oligonucleotides shown in Fig. 2 by a well-known method and then linking the 15 oligonucleotides fragments by a well-known method.

The DNA for the EC-SOD gene thus synthesized is cloned into a vector for cloning. As the vector for cloning, the one for the recombination of gene which has been constructed from phages or plasmids which can be proliferated autonomously in a microorganism as a host is preferred. As the former phages, λgt•λC or λgt•λB is used when the host microorganism is E. coli. As the plasmid, pBR322, pBR325, pACYC184, pUC12, pUC13, pUC18, pUC19, pUC118 or the like is used when the host microorganism is E. coli, or pUB110, pC194 or the like is used when Bacillus subtillis is used as the host microorganism. Furthermore, it is also possible to use a shuttle vector which is proliferated autonomously in two or more host microorganisms including Gram-negative and positive bacteria such as E. coli and Saccharomyces cerevisiae.

The methods for incorporating the DNA for the EC-SOD gene into these vectors are not limited, and the conventional method can be used. For example, recombinant DNA is obtained by cutting the vector with an appropriate restriction enzyme to obtain vector fragments, annealing the cohesive end of the vector fragment and the cohesive end of the DNA and then linking them with an appropriate DNA ligase.

The recombinant DNA thus obtained is transduced into a host microorganism for cloning the EC-SOD gene. As the host micoroorganism, there are no limitation except that it is the one in which the recombinant DNA is stable and capable of autonomous proliferation, and microorganisms conventionally used for the gene recombination such as E. coli is used preferably.

As the method for transducing the recombinant DNA in a host microorganism, for example the recombinant DNA is transduced in the presence of a calcium ion when the host microorganism is the one belonging to the Escherichia genus. When the host micoroorganism is the one belonging to the Bacillus genus, the competent cell method or the protoplast method is used or the microinjection method may also be used.

As for the selection of the transduction of the desired recombinant DNA into a host microorganism, host microorganisms are cultured in a selection medium based on the chemical-resistant marker of the vector constructing the recombinant DNA, and ones which are grown in the medium may be selected. Plasmid DNA containing the EC-SOD gene shown in the sequence number 1 of the sequence table is prepared from the transformed host microorganism according to the method of Maniatis et al.

In this connection, it is necessary for efficient expression of foreign protein gene in a transformed microorganism and for the large scale production of an aimed protein to enhance the transcriptional efficiency or the translational efficiency of the foreign protein gene or to increase the copy number of the foreign protein gene. Thus, in order to enhance the transcriptional efficiency of the foreign protein gene, linking of a strong promoter upstream the foreign protein gene is currently conducted.

The present inventors have previously found that the DNA fragment obtained by cutting the plasmid containing pyruvic acid oxidase with a restruction enzyme can express efficiently not only pyruvic acid

4

oxidase but also the genes of the other peptides or proteins such as SOD, and proposed an expression vector that the foreign protein gene is linked down stream the deoxyribonucleotide sequence containing the promoter which controls the expression of pyruvic acid oxidase (Japanese Patent Kokai No. 144976/1989).

Therefore, in the process of the present invention, a method for transducing an expression vector in which the EC-SOD gene has been integrated downstream the promoter is preferably used for the efficient production of EC-SOD in a large scale in a transformed microorganism.

Referring to an example of a preferred method for producing the expression vector, a promoter having about 200 bp is first cut out with restriction enzyme DraI from the plasmid pOX13 which is known as a preferred recombinant vector for expressing pyruvic acid oxidase gene, bonded to the plasmid pUC13 fragment cut with restriction enzyme SalI to make a promoter containing plasmid pKN19. The plasmid pKN19 possesses recognizing sites of restriction enzymes XbaI and EcoRI and thus are cut with these enzymes.

On the other hand, a plasmid DNA containing the EC-SOD gene prepared from aforementioned transformed host microorganism is cut with restriction enzymes XbaI and EcoRI to give a 700 bp EC-SOD fragment shown in the sequence number 1 of the sequence table, which is then bond to the plasmid pKN19 fragment to give an expression vector having the promoter as well as the EC-SOD gene downstream the promoter. Moreover, as an example of the other preferred methods for preparing expression vector, pIN-III-ompA which is known to be a preferred recombinant vector for excreting and expressing a desired polypeptide in the periplasm of E. coli may be used (The EMBO Journal, 3, 2437-2442 (1984)). In this case, it is sufficient that a DNA sequence corresponding to the fused polypeptide constructed by linking an ompA signal peptide which is consisted of from the initiating Met to Ala at the 21 position and a maturated polypeptide of EC-SOD is used. The polypeptide is cutted at a position just after Ala at the 21 position including initiating Met of the ompA signal peptide, and the maturated EC-SOD polypeptide is excreted and expressed.

The transduction of the expression vector thus obtained into a host microorganism and the transformation of the host microorganism will produce a transformed microorganism which produces EC-SOD. As the host microorganism used in this case, microorganisms used for conventional gene recombination such as protease-deleted E. coli or the like are preferably used. As for the transduction of the expression vector, there are no limitation, for example the recombinant DNA is transduced in the presence of a calcium ion when the host microorganism is the one belonging to the Escherichia genus. When the host microorganism is the one belonging to the Bacillus genus, the competent cell method or the protoplast method is used or the microinjection method may also be used.

As for the selection of the transduction of the expression vector into a host microorganism, host microorganisms are cultured in a selection medium based on the chemical-resistant marker of the vector, and ones which are grown in the medium may be selected.

The transformed microorganism thus obtained are cultured in a cuture medium containing nutrients required for the growth of the microorganism or inorganic components such as copper ion and zinc ion in a well-known method. When the microorganism is E. coli, culturing is performed at a temperature of 25 - 42°C, preferably about 37°C for 15 - 48 hours by the culturing methods such as aeration-agitation culture, shaking culture, rotary shaking culture, stationary culture or the like.

After culturing, the cells are collected by a centrifugation treatment, disrupted by the super-sonic treatment and then centrifuged to give the insoluble inactive aggregates containing recombinant EC-SOD which has no enzymatic activity as a protein fraction. The insoluble inactive aggregates containing recombinant EC-SOD is a precipitate obtained for example by the centrifugation treatment at 3,000 - 20,000 rpm for at least 5 minutes, usually at 3,000 rpm for about 10 - 30 minutes. It is an insoluble protein fraction which contains the aimed protein but exhibits no activity and is often called an inclusion body.

In the present invention, the insoluble inactive aggregates containing recombinant EC-SOD obtained by the centrifugation treatment at 3,000 - 20,000 rpm is first treated in a chaotropic environment under reducing conditions or in combination of the two, and the EC-SOD as a solubilized monomer is formed and recovered. As used herein, the term chaotropic environment refers to an environment which contains appropriate chaotropic agents, such as urea in sufficient concentration to disrupt the tertiary structure of proteins, or which is maintained at a temperature or other condition which causes such disruption. Chaotropic agents or conditions such as temperature and pH may disrupt structure in a variety of ways, including the disruption of hydrogen bonds. Suitable chaotropic environments include 4 - 10 M urea, preferably 6 - 8 M urea, 4 - 8 M guanidine detergents such as SDS at concentration around 0.1% by weight, and acids such as acetic acid at concentration of about 0.1 M, basic conditions of, e.g., pH 11 and above, and elevated temperatures. These chaotropic environments may be combined appropriately. For example, the insoluble inactive aggregates containing recombinant EC-SOD may be placed in an environment brought about by a chaotropic agent and then placed in the second chaotropic environment brought about by the other agents or temperature.

Also, the term reducing condition refers to a condition in which the insoluble inactive aggregates containing recombinant EC-SOD is maintained at a form reduced into a monomer or placed in such a condition. In order to bring about the condition, an appropriate reducing agent can be used. As the reducing agent, there are mentioned a SH reagent which is capable of reducing disulfide linkages to thiol groups such as 0 - 10% 2-mercaptoethanol, 0 - 100 mH dithiothreitol, glutathione and free cysteine.

As the chaotropic environment, the reducing conditions or in combination of the two, it is sufficient that the insoluble inactive forms of recombinant foreign proteins containing EC-SOD is left to stand at a concentration of 0.1 - 10 mg/ml of the aimed protein in a suitable buffer having a pH of 4 - 9, such as 10 - 100 mM of Tris-HCl buffer, phosphate buffer, acetate buffer or citrate buffer at a temperature of 4 - 40 °C for 1 - 40 hours. The insoluble inactive aggregates containing recombinant EC-SOD is thus converted into the monomeric EC-SOD and solubilized. In the present invention, the solubilized monomeric EC-SOD is recovered. On recovering the monomer, it is advantageous for the subsequent refolding processes to purify and recover the monomer with one or more of the purification means selected from size exclusion chromatography, ion exchange chromatography, affinity chromatography and hydrophobic interacting chromatography, according to necessities. The monomeric EC-SOD thus recovered is refolded into the active EC-SOD under the refolding conditions.

The term refolding conditions used herein refers to a condition in which the aforementioned solubilized monomeric EC-SOD is refolded into an active dimeric or polymeric structure by the formation of disulfide linkage or the dissociation. As the refolding conditions, there can be preferably mentioned a condition including a chaotropic environment in which disulfide linkage is formed by the air oxidation or a condition including a chaotropic environment in the presence of the oxidation-reduction system reagent. As the oxidation-reduction system reagent, there can be mentioned for example oxidized glutathione, reduced glutathione, an appropriate disulfide reagent (oxidized form) or a thiol reagent (reduced form). The oxidation-reduction system reagent preferably contains a concentration of 0 - 1 mM of the oxidized glutathione and 0 - 10 mM of the reduced glutathione. More preferably, the solution of the oxidation-reduction system reagent contains 0 - 0.05 mM of Cu ion- and Zn ion-releasing compound such as $CuSO_4$ or $ZnCl_2$. Furthermore, as the reactivation means used for the refolding conditions, there may be mentioned the dialysis method and the dilution-dialysiS method. As the dialysis method, the solution including monomeric EC-SOD may be dialyzed against 10 - 1000 fold of the appropriate buffer (pH 4 - 9) containing 0 - 10 mM of reduced glutathionet 0 - 1 mM of oxidized glutathione, 0 - 0.05 mM of $Cu^{++}$ and 0 - 0.05 mM of $Zn^{++}$ at 4 - 40°C for about 1 - 48 hours. Alternatively dialysis is made against 10 - 1000 fold of the same appropriate buffer (pH 4 - 9) as described above which contains a chaotropic reagent such as 0.5 - 4 M urea or 0.5 - 3 M guanisyl hydrochloride at 4 - 40°C for about 1 - 48 hours. Thereafter, further dialysis is made against 10 - 1000 fold of the appropriate buffer (pH 4 - 9) free from a chaotropic reagent under the same conditions as above.

The dilution-dialysis method is effected in such a manner that the solution including monomeric EC-SOD is diluted with 10 - 1000 fold of the appropriate buffer (pH 4 - 9) containing the chaotropic reagent, and dialyzed against the 1 - 1000 fold of appropriate buffer (pH 4 - 9) free from a chaotropic reagent under the same condition as above.

In the present invention, the active crude EC-SOD thus refolded is concentrated appropriately into 50 - 5000 times and subjected to a purification treatment in the subsequent step. As for the purification treatment, there is no limitation specifically, and any of conventionally well-known purification methods may be used, preferably size exclusion chromatography, ion exchange chromatography, affinity chromatography and hydrophobic interacting chromatography. These chromatographical methods may be used alone or in combination of the two or more. Alternatively, one or more purification methods may be used repeatedly. The enzymatic activity of the purified EC-SOD obtained by the purification treatment rises to 3000 unit/mg protein or more.

In the process of the present invention, the active EC-SOD in the form of 2 - 6 mer is obtained easily by the above-described method. The tetrameric EC-SOD having an especially high activity is produced by repeated purification.

In this connection, the marks in the base sequence of the EC-SOD gene and those in the amino acid sequences of proteins described in this specification are based on common abbreviations in the art, which are listed in the followings. All of the amino acids refers to L-isomers.

DNA:        deoxyribonucleic acid,
RNA:        ribonucleic acid,
mRNA:      messenger RNA,

6

cDNA:    complementary DNA,
A:    adenine,
T:    thymine,
G:    guanine,
C:    cytosine,
Ala:    alanine,
Arg:    arginine,
Asn:    asparagine,
Asp:    aspartic acid,
Cys:    cysteine,
Gln:    glutamine,
Glu:    glutamic acid,
Gly:    glycine,
His:    histidine,
Ile:    isoleucine,
Leu:    leucine,
Lys:    lysine,
Met:    methionine,
Phe:    phenylalanine,
Pro:    proline,
Ser:    serine,
Thr:    threonine,
Trp:    tryptophan,
Tyr:    tyrosine,
Val:    valine.

[Sequence table]

Sequence number:  1,

Length of sequence:  697 base pair,

Type of sequence:  nucleic acid,

Number of chain:  double strands,

Topology:  straight chain,

Kind of sequence:  cDNA to mRNA,

Origin:  Synthesis based on the one derived from human
      placenta,

Title of sequence:  EC-SOD gene,

Sequence:

```
CTAGAGGGTT ATTAATA ATG TGG ACT GGT GAA GAT TCT GCT GAA CCG AAC        50
                     Met Trp Thr Gly Glu Asp Ser Ala Glu Pro Asn
                      1               5                   10
```

```
TCT GAT TCT GCT GAA TGG ATC CGT GAT ATG TAC GCT AAA GTA ACT GAA        98
Ser Asp Ser Ala Glu Trp Ile Arg Asp Met Tyr Ala Lys Val Thr Glu
              15                  20                  25
```

```
ATC TGG CAG GAA GTT ATG CAG CGT CGT GAT GAC GAT GGT ACT CTG CAT        146
Ile Trp Gln Glu Val Met Gln Arg Arg Asp Asp Asp Gly Thr Leu His
          30                  35                  40
```

```
GCT GCT TGT CAG GTT CAG CCG TCT GCT ACT CTG GAC GCT GCA CAG CCA        194
Ala Ala Cys Gln Val Gln Pro Ser Ala Thr Leu Asp Ala Ala Gln Pro
          45                  50                  55
```

```
CGT GTT ACC GGT GTT GTT CTG TTC CGT CAG CTG GCA CCA CGT GCT AAA        242
Arg Val Thr Gly Val Val Leu Phe Arg Gln Leu Ala Pro Arg Ala Lys
          60                  65                  70
```

```
CTG GAT GCT TTC TTC GCT CTG GAA GGC TTC CCG ACT GAA CCG AAC TCT        290
Leu Asp Ala Phe Phe Ala Leu Glu Gly Phe Pro Thr Glu Pro Asn Ser
      75                  80                  85                  90
```

```
TCC TCT CGT GCT ATC CAT GTT CAT CAG TTC GGT GAT CTG TCT CAG GGT        338
Ser Ser Arg Ala Ile His Val His Gln Phe Gly Asp Leu Ser Gln Gly
                  95                  100                 105
```

```
TGT GAA TCT ACC GGT CCA CAT TAC AAC CCG CTG GCT GTT CCG CAC CCG        386
Cys Glu Ser Thr Gly Pro His Tyr Asn Pro Leu Ala Val Pro His Pro
              110                 115                 120
```

```
CAG CAT CCG GGT GAC TTC GGT AAC TTC GCG GTT CGT GAC GGC TCT CTG        434
Gln His Pro Gly Asp Phe Gly Asn Phe Ala Val Arg Asp Gly Ser Leu
          125                 130                 135
```

```
TGG CGT TAC CGT GCT GGT CTG GCT GCT TCT CTG GCG GGT CCG CAT TCC      482
Trp Arg Tyr Arg Ala Gly Leu Ala Ala Ser Leu Ala Gly Pro His Ser
    140             145             150


ATC GTT GGC CGT GCT GTT GTT GTT CAC GCT GGC GAA GAT GAC CTG GGT      530
Ile Val Gly Arg Ala Val Val Val His Ala Gly Glu Asp Asp Leu Gly
155             160             165             170


CGT GGT GGT AAC CAG GCT TCT GTT GAA AAC GGT AAC GCA GGC CGT CGT      578
Arg Gly Gly Asn Gln Ala Ser Val Glu Asn Gly Asn Ala Gly Arg Arg
            175             180             185


CTG GCT TGT TGC GTT GTT GGC GTT TGC GGT CCA GGT CTG TGG GAA CGT      626
Leu Ala Cys Cys Val Val Gly Val Gys Gly Pro Gly leu Trp Glu Arg
        190             195             200


CAG GCA CGT GAA CAT TCT GAA CGT AAA AAA CGT CGT CGT GAA TCC GAA      674
Gln Ala Arg Glu His Ser Glu Arg Lys Lys Arg Arg Arg Glu Ser Glu
        205             210             215


TGC AAA GCA GCT TGACAAGAAT T                                        697
Cys Lys Ala Ala
    220
```

Example

The present invention is now described in more detail with reference to Referenctial Examples and Examples without limit thereto.

Referential Example 1: Total synthesis of gene

Fifteen synthetic oligonucleotides shown in Fig. 2 were synthesized by the phosphoamidite method with an automatic DNA synthesizer (manufactured by BIOSEARCH CO., CYCLONE PLUS DNA SYNTHESIZER). The 1 $\mu$g portions of the fifteen synthetic nucleotides were mixed together, and 50 mM Tris-HC1 (pH 7.6), 10 mM MgCl$_2$, 10 mM dithiothreitol, 0.1 mM spermidine, 0.1 mM EDTA, 1 mM APT and 10 units of T4 polynucleotide kinase (manufactured by TAKARA SHUZO) were added to the mixture, and the total amount was adjusted to 50 $\mu$l. After the mixture was left to stand at 37°C for 30 minutes, an equivalent amount of phenol-chloroform was added, agitated and centrifuged at 10,000 rpm for 3 minutes and the aqueous layer was separated.

Then, the 1/10 equivalent amount of a 3 M sodium acetate solution and the two equivalent amount of ethanol were added to the aqueous layer. After the mixture was left standing at -80°C for 15 minutes and centrifuged at 15,000 rpm for 15 minutes, precipitates were collected and dried under reduced pressure (these operations are referred to hereinafter as phenol extraction treatment and ethanol precipitation treatment). The precipitates were dissolved in a solution containing 0.1 M NaCl and 15 $\mu$l of TE, i.e., a mixture of 10 mM Tris-HCl and 1 mM EDTA, heated at 100°C for 5 minutes and left standing at room temperature for 2 hours.

Then, 50 mM Tris-HC1 (pH 7.6), 10 mM $MgCl_2$, 10 mM dithiothreitol, 0.1 mM spermidine, 0.1 mM EDTA, 1 mM ATP and 200 units of T4 ligase were added to the solution, and the mixture was adjusted to the total amount of 100 $\mu$l and ligated at 14°C for 18 hours. The reaction liquor thus obtained was subjected to the phenol treatment and the ethanol treatment in the same manner as described above to give precipitates.

To the precipitates were then added the X10H buffer (manufactured by TAKARA SHUZO CO.) and 100 units each of the restriction enzymes XbaI and EcoRI. The mixture, after being adjusted to the total amount of 100 $\mu$l, was digested at 37°C for 2 hours. The reaction liquor was subjected to the phenol treatment and the ethanol treatment to give precipitates.

On the other hand, 1 $\mu$g of pUC118 plasmid DNA (manufactured by TAKARA SHUZO CO.) was digested in 10 $\mu$l of a solution containing the X10H buffer (ditto) and 5 units each of the restriction enzymes XbaI and EcoRI at 37°C for 2 hours. Then, 1 unit of bacteria alkaline phosphatase (manufactured by TAKARA SHUZO CO.) was added, and the mixture was reacted at 65°C for 1 hour. The reaction liquor was subjected to the phenol treatment and the ethanol treatment to give precipitates.

The pUC118 vector and the previously obtained precipitates which were the linked product of the fifteen synthetic nucleotides were subjected to ligation reaction with 200 units of T4 ligase.

Meanwhile, E. coli MV1184 (purchased from TAKARA SHUZO CO.) at the logarithmic proliferation phase which had been cultured in 100 ml of LB medium containing 10 g of bacto-trypton, 5 g of bacto-yeast extract and 10 g of NaCl per 1 liter was collected, suspended into 40 ml of an ice-cooled solution (pH 5.8) containing 30 mM potassium acetate, 100 mM RbCl, 10 mM $CaCl_2$, 50 mM $MnCl_2$ and 15% by weight of glycerol, left standing at 0°C for 5 minutes, collected again by centrifugation and suspended into 4 ml of a solution (pH 6.5) containing 10 mM MOPS, 75 MM $CaCl_2$, 10 mM RbCl and 15% by weight of glycerol to make competent cells. To a 200 $\mu$l portion of the E. coli suspension, 20 $\mu$l of the ligated DNA solution was added, and the mixture was left standing at 0°C for 30 minutes. Then, after 800 $\mu$l of the LB medium was added, the mixture was maintained at a temperature of 37°C for 1 hour, inoculated on an LB agar plate containing 50 $\mu$g/ml of ampicillin and cultured overnight to give transformed E. coli MV1184 pUC118-sEC-SOD.

Plasmid DNA was next prepared from the transformed E. coli MV1184 pUC118-sEC-SOD thus obtained according to the method of Maniatis et al. (Molecular Cloning, 1982, Cold Spring Harbor Laboratory). A 1 $\mu$g portion of the pUC118-sEC-SOD plasmid DNA was digested in a solution containing the X10H buffer (ditto) and 5 units each of restriction enzymes XbaI and EcoRI at 37°C for 2 hours and fractionated on a 1% agarose gel to confirm the insertion of DNA of 700 bp. Furthermore, the total sequence of the XbaI-EcoRI DNA insertion fragment was determined by use of M-13 sequence kit (TAKARA SHUZO CO.). The sequence is shown in the sequence number 1 of the sequence table.

Referential Example 2 Construction of expression vector

A 20 $\mu$g portion of the pUC118-sEC-SOD plasmid DNA obtained in Referential Example 1 was digested in 100 $\mu$l of a solution containing the X10H buffer and 40 units each of restriction enzymes XbaI and EcoRI at 37°C for 2 hours and fractionated on a 1% low-melting agarose to isolate a DNA fragment of about 700 bp.

Also, a 10 $\mu$g portion of the pOXI3 plasmid DNA prepared from the E. coli W3110 pOXI3 (FERMBP-1565) strain deposited at FERM bearing deposition No. FERM P9071, on December 8, 1986 and changed to a deposition under Budapest Treaty on November 9, 1987, bearing deposition No. BP 1565, was digested in 10 $\mu$l of a solution containing the X10H buffer (manufactured by TAKARA SHUZO CO.) and 20 units of the restriction enzyme DraI at 37°C for 2 hours and fractionated on a 1% low-melting agarose to isolate a DNA fragment of about 200 bp.

Meanwhile, 1 $\mu$g of the pUC13 plasmid DNA (manufactured by TAKARA SHUZO CO.) was digested in 10 $\mu$l of a solution containing the X10H buffer (ditto) and 5 units of the restriction enzyme SalI at 37°C for 2 hours and subjected to the phenol extraction treatment and the ethanol precipitation treatment to give precipitates. The precipitates were dissolved in 10 $\mu$l of liquor containing 67 mM $KPO_4$, 6.7 mM $MgCl_2$, 1 mM 2-mercaptoethanol and 33 $\mu$M dATP, dCTP, dGTP and dTTP. Klenow fragment enzyme (manufactured by TAKARA SHUZO CO, 20 units) was added to the liquor, and the mixture was reacted at 22°C for 1 hour. To the mixture, 1 unit of bacteria alkaline phosphatase was added, and the mixture was treated according to the method described above.

One hundred ng of the ca. 200 bp DNA fragment recovered from the agarose gel and 50 ng of the pUC13 DNA fragment obtained were ligated with 200 units of T4 ligase in the same manner as described above. The reaction liquor was added to 200 μl of the E. coli ME8417 competent cell and left standing at 4°C for 30 minutes. Then, 800 μl of LB medium was added, and the mixture was maintained at 37°C for 1 hour, inoculated on an LB agar plate containing 50 μg/ml of ampicillin and cultured overnight to give the transformed E. coli ME8417 pKN19 (FERMBP-3204 deposited at FERM under the Budapest Treaty on December 14, 1990).

One μg of the pKN19 plasmid DNA prepared from the transformant was digested in 10 μl of a solution containing the X10H buffer and 5 units each of the restriction enzymes XbaI and EcoRI at 37°C for 2 hours and then treated with 1 unit of bacteria alkaline phosphatase in the same manner as described above. Then, 100 ng of the ca. 700 bp sEC-SOD DNA fragment recovered from the agarose gel and 50 ng of the XbaI, EcoRI-digested pKN19DNA were ligated with 200 units of T4 ligase in the same manner as described above. The liquor obtained was then added to 200 μl of the competent cell prepared from the E. coli ME8417 strain in the same manner as described above, and the mixture was left standing at 4°C for 30 minutes. Then, 800 μl of the LB medium wad added, and the mixture was maintained at a temperature of 37°C for 1 hour, inoculated on an LB agar plate containing 50 μg/ml of ampicillin and cultured overnight to give the transformed E. coli ME8417 pKN19-sEC-SOD (FERMBP-3205 deposited at FERM under the Budapest Treaty on December 14, 1990). Plasmid DNA was prepared from the transformant thus obtained and digested with the restriction enzymes XbaI and EcoRI, and an inserted DNA fragment of ca. 700 bp was confirmed by electrophoresis on 1% agarose gel.

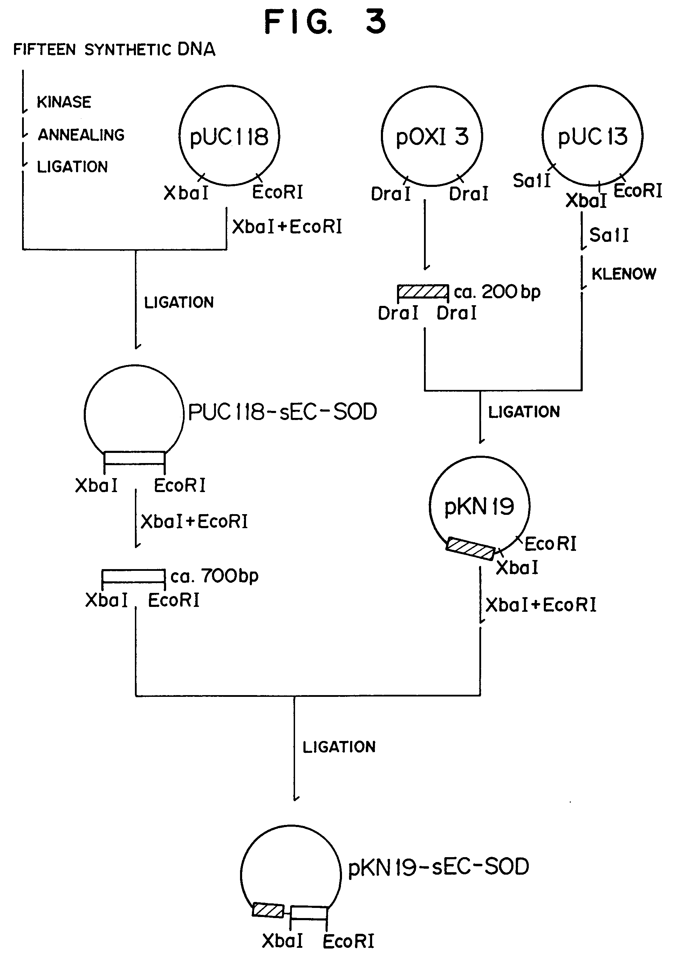

The flow chart of the construction of the EC-SOD expression vector by the methods in Referential Examples 1 and 2 are illustrated in Fig. 3.

Referential Example 3 Expression of EC-SOD

The transformant E. coli ME8417 pKN19-sEC-SOD obtained in Referential Example 2 was subjected to shaking culture in 10 ml of an LB medium containing 50 μg/ml of ampicillin at 37°C for 18 hours and then centrifuged at 3,000 rpm for 10 minutes to give the cell pellet. The pellet was suspended into 1 ml of a solution comprising 50 mM Tris-HC1 (pH 7.6), 50 mM sucrose, 1 mM CuSO₄ and 1 mM ZnCl₂ and disrupted by a supersonic treatment at 4°C for 1 minute.

At the same time, E. coli ME8417 pKN19 as a control was cultured in the same manner as described above, and then the cells were disrupted.

Ten μl each of the disrupted cell liquors was subjected to SDS-PAGE in an acrylamide concentration of 12.5% by weight according to the method of Lamemmli (Nature, 227, 680 (1970)). The CBB (Coomassie brilliant blue) staining of the gel revealed a band in the neighborhood of a protein having a molecular weight of ca. 28 Kd for the transformant E. coli ME8417 pKN19-sEC-SOD, said band being not observed in the control. When the band was determined quantitatively with a gel scanner (manufactured by Shimadzu Seisakusho, Ltd.), it occupied 20% by weight based on the total cell protein.

When the SOD enzymatic activities of these cell disrupted liquors were measured by the NBT method (Anal. Biochem., 44, 276-287 (1971)), both the transformant E. coli ME8417 pKN19-sEC-SOD and the control E. coli ME8417 pKN19 exhibited SOD activity of only 10 units/ml. It was recognized from the result that the activities of the two liquors show those derived from E. coli.

Furthermore, when the disrupted cell liquor of the transformant E. coli ME8417 pKN19-sEC-SOD was centrifuged at 3,000 rpm for 10 minutes and separated into a precipitate fraction and a soluble fraction. Each of the fractions was subjected to SDS-PAGE and then to the CBB staining, a band corresponding to EC-SOD having a molecular weight of 28 Kd was detected only from the precipitate fraction.

It was found from these results that the EC-SOD protein produced by the transformant E. coli ME8417 pKN19-sEC-SOD was the EC-SOD protein of the insoluble inactive forms of recombinant foreign proteins containing EC-SOD, that is, the inclusion body.

Example 1

(1) Separation, monomerization and reactivation of the insoluble product

The transformant E. coli ME8417 pKN19-sEC-SOD obtained in Referential Example 2 was shaking-cultured in 1 liter of an LB medium containing 50 μg/mg of ampicillin at 37°C for 18 hours and centrifuged at 5,000 rpm for 10 minutes to give a cell pellet. The pellet was then suspended in 20 ml of a solution comprising 50 mM Tris-HC1 (pH 7.5), 50 mM sucrose, 1 mM CuSO₄ and 1 mM ZnCl₂, disrupted by a supersonic treatment at 4°C for 5 minutes and centrifuged at 10,000 rpm for 10 minutes to give precipitates (referred to hereinafter as insoluble EC-SOD).

A 20 mg portion of the insoluble EC-SOD protein was incubated in 20 ml of a solution comprising 8 M urea, 1 mM dithiothreitol, 50 mM sucrose and 50 mM Tris-HC1 (pH 7.5) at 4°C for 1 hour and centrifuged at 40,000 rpm for 30 minutes to remove insolubilized products. The supernatant was then diluted by adding dropwise to a solution (2 liter) comprising 4 M urea, 1 mM reduced glutathione, 0.1 mM oxidized glutathione, 50 mM sucrose and 50 mM Tris-HC1 (pH 7.5) and incubated with stirring at 4°C for 18 hours.

Subsequently, the dilution was charged in a dialysis membrane and dialyzed thrice against 20 liters of a 10 mM Tris-HC1 (pH 7.5) at 4°C for 6 hours per dialysis. The dialyzed internal liquor was centrifuged at 5,000 rpm for 30 minutes to remove precipitates, concentrated to about 100 times with a LABO CASSETTE (trade name, manufactured by MILLIPORE CO.) and further concentrated to about 20 times with a CENTRIPREP (trade name, manufactured by AMICON CO.) to give 1 ml of a crude product. The crude product contained protein in an amount of 13.5 mg. The sample had an SOD activity of 100 units/mg protein according to the aforementioned NBT method.

### (2) Separation and purification of the crude product

A 200 $\mu$l portion of the EC-SOD crude product obtained in (1) was placed on a gel permeation chromatography column TSKge1G3000SW$_{XL}$ (7.8 mm x 30 cm, manufactured by TOSO CO.) which had been equilibrated with 50 mM Tris-HC1 (pH 7.0) containing 0.2 M NaCl and developed with the same developing solution as above. The SOD activities of respective fractions were measured by the NBT method, and the active fractions at 8 - 9 ml of elution were collected. This operation was repeated further four times. The collected fractions contained protein in an amount of 4.5 mg and showed the SOD enzymatic activity of 260 units/mg protein.

NaCl was removed from the active fraction with a desalting column PD-10 (manufactured by Pharmacia Co.) and the active fraction was then placed and adsorbed on an anion exchange resin column Mono-Q which had been equilibrated with 10 mM Tris-HC1 (pH 7.0) (5 mm x 5 cm, manufactured by Pharmacia Co.). After the column was washed with the same buffer, development was conducted with a linear gradient of 0 - 1 M NaCl in the buffer solution. The SOD activities in respective fractions were measured by the NBT method, and the active fractions eluted with NaCl concentrations of 0.3 - 0.6 M were collected. The collected fractions contained protein in an amout of 1.1 mg and showed the SOD enzymatic activity of 1104 units/mg protein.

Subsequently, after NaCl was removed from the active fraction with a desalting column PD-10 (manufactured by Pharmacia Co.), the Tris-HC1 buffer was substituted by 50 mM phosphate buffer (pH 7.0) containing 1.8 M ammonium sulfate. The active fraction was then placed and adsorbed on a phenyl-Superose column which had been equilibrated with a solution having the same composition as above (5 mm x 5 cm, manufactured by Pharmacia Co.). After the column was washed with the same buffer, development was conducted with a linear gradient of 1.8 - 0 M ammonium sulfate in 50 mM phosphate buffer (pH 7.0). The SOD activities in respective fractions were measured by the NBT method, and the active fractions eluted with ammonium sulfate concentrations of 1 - 0.7 M were collected. The collected fractions contained protein in an amount of 0.3 mg and exhibited the SOD enzymatic activity of 3050 units/mg protein.

When the fraction corresponding to 10 $\mu$g of protein was analyzed by an automatic amino acid sequencer (PSG-1 model, Shimadzu Seisakusho, Ltd.), amino acid sequence at the N-end was Trp-Thr-Gly-Glu-Asp-Ser-Ala-Glu-Pro-Asn-, which coincided with the amino acid sequence at the N-end of EC-SOD.

The fraction corresponding to 10 $\mu$g of protein was placed on the gel permeation chromatography column TSKge1G3000SW$_{XL}$ (ditto) which had been equilibrated with 50 mM Tris-HC1 (pH 7.0) containing 0.2 M NaCl and developed in the same manner as described above. When the elution pattern was measured at OD$_{280}$ nm, a peak was observed in the neighborhood of a molecular weight of 130 Kd.

Furthermore, when the fraction corresponding to 1 $\mu$g of protein was analyzed by SDS-PAGE (ditto), a single band was observed in the neighborhood of a molecular weight of 28 Kd. When the fraction corresponding to 200 $\mu$g of protein was analyzed with an atomic absorption spectrometer (AA-680/G, Shimadzu Seisakusho, Ltd.), substantially equivalent molar amounts of Cu and Zn ions per monomer were detected.

From these results, the Cu, Zn type tetrameric EC-SOD which comprises four monomers having a molecular weight of ca. 28 Kd and has a molecular weight of ca. 130 Kd was successfully separated and purified.

Example 2

After the transformant E. coli ME8417 pKN19-sEC-SOD obtained in Referential Example 2 was shaking-cultured in 1 liter of an LB medium containing 50 $\mu$g/mg of ampicillin at 37°C for 18 hours, it was centrifuged at 5,000 rpm for 10 minutes to obtain a cell pellet. The pellet was suspended in 20 ml of a solution comprising 50 mM Tris-HCl (pH 7.5), 50 mM sucrose, 1 mM CuSO$_4$ and 1 mM ZnCl$_2$, disrupted by a supersonic treatment at 4°C for 5 minutes and then centrifuged at 10,000 rpm for 10 minutes to give the insoluble EC-SOD.

A 2 mg portion of the insoluble EC-SOD was incubated in a solution (2 ml) comprising 6 M guanidine hydrochloride, 1 mM dithiothreitol, 50 mM sucrose and 50 mM Tris-HCl (pH 7.5) at 4°C for 1 hour and centrifuged at 40,000 rpm for 30 minutes to remove insolubilized products. The supernatant was then diluted by adding dropwise under stirring to 200 ml of a solution comprising 4 M urea, 1 mM reduced glutathione, 0.1 mM oxidized glutathione, 50 mM sucrose and 50 mM Tris-HC1 (pH 7.5) and incubated at 4°C for 18 hours.

Subsequently, the dilution was charged in a dialysis membrane and dialyzed thrice against 2 liters of a 10 mM Tris-HC1 (pH 7.5) at 4°C for 4 hours per dialysis, and the dialyzed internal liquor was centrifuged at 5,000 rpm for 30 minutes to remove precipitates, concentrated to about 50 times with a LABO CASSETTE (trade name, manufactured by MILLIPORE CO.) and further concentrated to about 20 times with a CENTRIPREP (trade name, manufactured by AMICON CO.) to give 0.5 ml of a crude product.

Subsequently, purification was conducted in the same manner as in Example 1, an enzymatically active EC-SOD (3080 units/mg protein) was obtained in an amount of 0.025 mg.

Example 3

After the transformant E. coli ME8417 pKN19-sEC-SOD obtained in Referential Example 2 was shaking-cultured in 1 liter of an LB medium containing 50 $\mu$g/mg of ampicillin at 37°C for 18 hours, it was centrifuged at 5,000 rpm for 10 minutes to obtain a cell pellet. The pellet was suspended in 20 ml of a solution comprising 50 mM Tris-HCl (pH 7.5), 50 mM sucrose, 1 mM CuSO$_4$ and 1 mM ZnCl$_2$, disrupted by a supersonic treatment at 4°C for 5 minutes and then centrifuged at 10,000 rpm for 10 minutes to give the insoluble EC-SOD.

A 2 mg portion of the insoluble EC-SOD was incubated in 2 ml of a solution comprising 8 M urea, 1 mM dithiothreitol, 50 mM sucrose and 50 mM Tris-HCl (pH 7.5) at 4°C for 1 hour and centrifuged at 40,000 rpm for 30 minutes to remove insolubilized products. The supernatant was then charged in a dialysis membrane and dialyzed twice against a solution (400 ml) comprising 4 M urea, 1 mM reduced glutathione, 0.1 mM oxidized glutathione, 50 mM sucrose and 50 mM Tris-HCl (pH 7.5) at 4°C for 8 hours per dialysis.

Subsequently, the dialyzed internal liquor was further dialyzed thrice against 2 liters of a 10 mM Tris-HCl (pH 7.5) solution, centrifuged at 5,000 rpm for 30 minutes to remove precipitates, concentrated to about 4 times with CENTRIPREP (trade name, manufactured by AMICON CO.) to give 0.5 ml of a crude product.

Subsequently, purification was conducted in the same manner as in Example 1, an enzymatically active EC-SOD (3010 units/mg protein) was obtained in an amount of 0.021 mg.

Example 4

After the transformant E. coli ME8417 pKN19-sEC-SOD obtained in Referential Example 2 was shaking-cultured in 1 liter of an LB medium containing 50 $\mu$g/mg of ampicillin at 37°C for 18 hours, it was centrifuged at 5,000 rpm for 10 minutes to obtain a cell pellet. The pellet was suspended in 20 ml of a solution comprising 50 mM Tris-HCl (pH 7.5), 50 mM sucrose, 1 mM CuSO$_4$ and 1 mM ZnCl$_2$, disrupted by a supersonic treatment at 4°C for 5 minutes and then centrifuged at 10,000 rpm for 10 minutes to give insoluble EC-SOD.

A 2 mg portion of the insoluble EC-SOD was incubated in 2 ml of a solution comprising 8 M urea, 2% 2-mercaptoethanol, 50 mM sucrose and 50 mM Tris-HCl (pH 7.5) at 4°C for 1 hour and centrifuged at 40,000 rpm for 30 minutes to remove insolubilized products. The supernatant was then diluted by adding dropwise under stirring to 200 ml of a solution comprising 4 M urea, 1 mM reduced glutathione, 0.1 mM oxidized glutathione, 50 mM sucrose and 50 mM Tris-HCl (pH 7.5) and incubated at 4°C for 18 hours.

Subsequently, the dilution was charged in a dialysis membrane and dialyzed thrice against 2 liters of a 10 mM Tris-HCl (pH 7.5) containing 0.1 mM oxidized gultathione, at 4°C for 6 hours per dialysis, and the dialyzed internal liquor was centrifuged at 5,000 rpm for 30 minutes to remove precipitates, concentrated to about 50 times with a LABO CASSETTE (trade name, manufactured by MILLIPORE CO.) and further concentrated to about 20 times with a CENTRIPREP (trade name, manufactured by AMICON CO.) to give 0.5 ml of a crude product.

Subsequently, purification was conducted in the same manner as in Example 1, an enzymatically active EC-SOD (3005 units/mg protein) was obtained in an amount of 0.022 mg.

**Claims**

1. A process for producing enzymatically active purified EC-SOD from insoluble inactive aggregates containing recombinant EC-SOD, which comprises sequentially performing the following steps:

   (a) the insoluble inactive aggregates containing recombinant EC-SOD are treated under a chaotropic environment or a reducing condition until the EC-SOD in the form of a solubilized monomer is formed and recovered;

   (b) the monomeric EC-SOD recovered in the step (a) is refolded into an active EC-SOD under a refolding condition; and

   (c) the active EC-SOD refolded in the step (b) is purified.

2. A production process according to claim 1, wherein the step (b) is a step of refolding under the refolding conditions including a chaotropic environment in which a disulfide linkage is formed by air oxidation.

3. A production process according to claim 1, wherein the step (b) is a step of refolding under the refolding conditions including a chaotropic environment in the presence of an oxidation-reduction reagent.

4. A production process according to any one of claims 1 - 3, wherein the step (c) is a step of purification with use of at least a chromatography selected from size exclusion chromatography, ion exchange chromatography, affinity chromatography and hydrophobic interacting chromatography.

5. A production process according to any one of claims 1 - 4, wherein the active extracellular superoxide dismutase is a 2 - 6 mer.

6. A production process according to claim 5, wherein the active extracellular superoxide dismutase is a tetramer.

14

# FIG. 1-1

```
CTAGAGGGTTATTAATAATGTGGACTGGTGAAG
    TCCCAATAATTATTACACCTGACCACTTC

ATTCTGCTGAACCGAACTCTGATTCTGCTGAAT
TAAGACGACTTGGCTTGAGACTAAGACGACTTA

GGATCCGTGATATGTACGCTAAAGTAACTGAAA
CCTAGGCACTATACATGCGATTTCATTGACTTT

TCTGGCAGGAAGTTATGCAGCGTCGTGATGACG
AGACCGTCCTTCAATACGTCGCAGCACTACTGC

ATGGTACTCTGCATGCTGCTTGTCAGGTTCAGC
TACCATGAGACGTACGACGAACAGTCCAAGTCG

CGTCTGCTACTCTGGACGCTGCACAGCCACGTG
GCAGACGATGAGACCTGCGACGTGTCGGTGCAC

TTACCGGTGTTGTTCTGTTCCGTCAGCTGGCAC
AATGGCCACAACAAGACAAGGCAGTCGACCGTG

CACGTGCTAAACTGGATGCTTTCTTCGCTCTGG
GTGCACGATTTGACCTACGAAGAAGCGAGACC

AAGGCTTCCCGACTGAACCGAACTCTTCCTCTC
TTCCGAAGGGCTGACTTGGCTTGAGAAGGAGAG

GTGCTATCCATGTTCATCAGTTCGGTGATCTGT
CACGATAGGTACAAGTAGTCAAGCCACTAGACA

CTCAGGGTTGTGAATCTACCGGTCCACATTACA
GAGTCCCAACACTTAGATGGCCAGGTGTAATGT
```

# FIG. 1-2

```
ACCCGCTGGCTGTTCCGCACCCGCAGCATCCGG
TGGGCGACCGACAAGGCGTGGGCGTCGTAGGCC

GTGACTTCGGTAACTTCGCGGTTCGTGACGGCT
CACTGAAGCCATTGAAGCGCCAAGCACTGCCGA

CTCTGTGGCGTTACCGTGCTGGTCTGGCTGCTT
GAGACACCGCAATGGCACGACCAGACCGACGAA

CTCTGGCGGGTCCGCATTCCATCGTTGGCCGTG
GAGACCGCCCAGGCGTAAGGTAGCAACCGGCAC

CTGTTGTTGTTCACGCTGGCGAAGATGACCTGG
GACAACAACAAGTGCGACCGCTTCTACTGGACC

GTCGTGGTGGTAACCAGGCTTCTGTTGAAAACG
CAGCACCACCATTGGTCCGAAGACAACTTTTGC

GTAACGCAGGCCGTCGTCTGGCTTGTTGCGTTG
CATTGCGTCCGGCAGCAGACCGAACAACGCAAC

TTGGCGTTTGCGGTCCAGGTCTGTGGGAACGTC
AACCGCAAACGCCAGGTCCAGACACCCTTGCAG

AGGCACGTGAACATTCTGAACGTAAAAAACTGC
TCCGTGCACTTGTAAGACTTGCATTTTTGACG

GTCGTGAATCCGAATGCAAAGCAGCTTGACAAG
CAGCACTTAGGCTTACGTTTCGTCGAACTGTTC


TTAA
```

# FIG. 2-1

No.1 (100)
CTAGAGGGTT ATTAATAATG TGGACTGGTG AAGATTCTGC TGAACCGAAC TCTGATTCTG CTGAATGGAT
CCGTGATATG TACGCTAAAG TAACTGAAAT

No.2 (100)
CTGGCAGGAA GTTATGCAGC GTCGTGATGA CGATGGTACT CTGCATGCTG CTTGTCAGGT TCAGCCGTCT
GCTACTCTGG ACGCTGCACA GCCACGTGTT

No.3 (100)
ACCGGTGTTG TTCTGTTCCG TCAGCTGGCA CCACGTGCTA AACTGGATGC TTTCTTCGCT CTGGAAGGCT
TCCCGACTGA ACCGAACTCT TCCTCTCGTG

No.4 (100)
CTATCCATGT TCATCAGTTC GGTGATCTGT CTCAGGGTTG TGAATCTACC GGTCCACATT ACAACCCGCT
GGCTGTTCCG CACCCGCAGC ATCCGGGTGA

No.5 (100)
CTTCGGTAAC TTCGCGGTTC GTGACGGCTC TCTGTGGCGT TACCGTGCTG GTCTGGCTGC TTCTCTGGCG
GGTCCGCATT CCATCGTTGG CCGTGCTGTT

No.6 (100)
GTTGTTCACG CTGGCGAAGA TGACCTGGGT CGTGGTGGTA ACCAGGCTTC TGTTGAAAAC GGTAACGCAG
GCCGTCGTCT GGCTTGTTGC GTTGTTGGCG

No.7 (93)
TTTGCGGTCC AGGTCTGTGG GAACGTCAGG CACGTGAACA TTCTGAACGT AAAAAACGTC GTCGTGAATC
CGAATGCAAA GCAGCTTGAC AAG

EP 0 492 447 A1

## FIG. 2-2

No.8 (46)

GTTCGGTTCA GCAGAATCTT CACCAGTCCA CATTATTAAT AACCCT

No.9 (100)

CAGCATGCAG AGTACCATCG TCATCACGAC GCTGCATAAC TTCCTGCCAG ATTTCAGTTA CTTTAGCGTA
CATATCACGG ATCCATTCAG CAGAATCAGA

No.10 (100)

GCATCCAGTT TAGCACGTGG TGCCAGCTGA CGGAACAGAA CAACACCGGT AACACGTGGC TGTGCAGCGT
CCAGAGTAGC AGACGGCTGA ACCTGACAAG

No.11 (100)

GGTAGATTCA CAACCCTGAG ACAGATCACC GAACTGATGA ACATGGATAG CACGAGAGGA AGAGTTCGGT
TCAGTCGGGA AGCCTTCCAG AGCGAAGAAA

No.12 (100)

CAGCACGGTA ACGCCACAGA GAGCCGTCAC GAACCGCGAA GTTACCGAAG TCACCCGGAT GCTGCGGGTG
CGGAACAGCC AGCGGGTTGT AATGTGGACC

No.13 (100)

GAAGCCTGGT TACCACCACG ACCCAGGTCA TCTTCGCCAG CGTGAACAAC AACAGCACGG CCAACGATGG
AATGCGGACC CGCCAGAGAA GCAGCCAGAC

No.14 (100)

ACGTTCAGAA TGTTCACGTG CCTGACGTTC CCACAGACCT GGACCGCAAA CGCCAACAAC GCAACAAGCC
AGACGACGGC CTGCGTTACC GTTTTCAACA

No.15 (47)

AATTCTTGTC AAGCTGCTTT GCATTCGGAT TCACGACGAC GTTTTTT

EP 0 492 447 A1

EP 0 492 447 A1

# FIG. 3

19

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WORLD PATENTS INDEX LATEST<br>Week 3788,<br>Derwent Publications Ltd., London, GB;<br>AN 88-262065<br>& JP-A-63 192 384 (AJINOMOTO KK) 9 August 1988<br>* abstract *<br>--- | 1,3,4 | C12N9/02<br>C12P21/00 |
| X | WORLD PATENTS INDEX LATEST<br>Week 4187,<br>Derwent Publications Ltd., London, GB;<br>AN 85-041685<br>& JP-A-60 001 295 (PIAS KK) 7 January  1985<br>* abstract *<br>--- | 1,3,4 | |
| A | EP-A-0 218 480 (SUNTORY LTD.)<br>* page 3, line 15 - line 21 *<br>* page 7, line 22 - line 31 *<br>----- | 1,5,6 | TECHNICAL FIELDS<br>SEARCHED (Int. Cl.5)<br><br>C12N |

The present search report has been drawn up for all claims

| Place of search | Date of completion of the search | Examiner |
|---|---|---|
| BERLIN | 17 MARCH 1992 | GURDJIAN D. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................................
& : member of the same patent family, corresponding
    document

EPO FORM 1503 03.82 (P0401)